# FASCICULE DE BREVET EUROPEEN

(11) **EP 1 545 632 B1**
(45) Date de publication et mention de la délivrance du brevet: **23.09.2009**
(21) Numéro de dépôt: 02807609.9
(22) Date de dépôt: 19.07.2002
(51) Int. Cl.: A61L 9/03, B60H 3/00, B60N 3/14

(54) **PROCEDE ET DISPOSITIF DE DIFFUSION D'AU MOINS UN PRODUIT VOLATIL DANS UN HABITACLE DE VEHICULE**
VERFAHREN UND VORRICHTUNG FÜR DIE DIFFUSION MINDESTENS EINES FLÜCHTIGEN PRODUKTS IN EINER FAHRZEUGKABINE
METHOD AND DEVICE FOR DIFFUSING AT LEAST ONE VOLATILE PRODUCT IN A VEHICLE PASSENGER COMPARTMENT

(43) Date de publication de la demande: 29.06.2005
(73) Titulaire: Bouthiaux, Catherine, 33000 Bordeaux (FR)
(72) Inventeur: Bouthiaux, Catherine, 33000 Bordeaux (FR)
(74) Mandataire: Fantin, Laurent
(86) Numéro de dépôt international: PCT/FR2002/002590
(87) Numéro de publication internationale: WO 2004/009141

(56) Documents cités:
- EP-A- 0 773 703
- WO-A-97/32611
- DE-A- 19 934 380
- FR-A- 2 727 630
- GB-A- 2 062 199
- US-A- 4 692 590
- US-A- 4 731 521
- US-A- 6 085 027
- US-B1- 6 249 645

## Description

La présente invention concerne un dispositif de diffusion d'au moins un produit volatil, notamment un parfum, dans un habitacle de véhicule. Dans les véhicules, l'habitacle est un lieu clos de faible volume. Bien qu'il existe des systèmes de renouvellement et de traitement d'air, le temps de renouvellement est relativement long.

De ce fait, toute odeur générée dans l'habitacle ou issue de l'extérieur lors du renouvellement, stagne pendant une période suffisante pour être incommodante pour les passagers.

Ainsi, la fumée de cigarette subsiste durant toute la durée de consommation de la cigarette et bien au-delà compte tenu de la ténacité de l'odeur, et plus encore lorsqu' il s'agit de certains autres produits comme les cigares.

De façon générale, on connaît des diffuseurs de parfum destinés à masquer ces odeurs qui se présentent sous la forme de plaquettes imbibées d'un produit parfumé à faible volatilité.

Ainsi, la libération se prolonge pendant une période donnée.

On a noté néanmoins que, au début, la puissance de masquage est grande, voire souvent trop forte et désagréable mais qu'ensuite, elle diminue très fortement après une brève période, rendant ce type de diffuseur inefficace.

Ceci est inéluctable et relève du principe même utilisé qui n'est pas commandé par de quelconques moyens par l'utilisateur, mais auto-diffusant.

Certains moyens ont été mis en place comme des boîtiers avec un flux de passage d'air réglable mais cela dépend aussi de la qualité de l'air sec ou humide et il faut penser à régler systématiquement l'ouverture pour ajuster les effets.

D'autres moyens plus simples consistent simplement à dégager plus ou moins une plaquette de son emballage. Ceci est d'une part mal aisé, difficilement contrôlable et de plus il subsiste l'emballage ouvert autour de la plaquette, puisqu'il agit comme moyen de réglage, ce qui est non esthétique.

Dans tous les cas, on note la présence d'une plaquette ou d'un boîtier ajouté sur le tableau de bord ou à portée de main pour le moins.

Un autre inconvénient de ces produits est celui d'une diffusion permanente. De deux choses l'une, soit les passagers s'habituent et cela ne confère plus les propriétés recherchées de masquage au moment opportun, soit la puissance est trop faible.

On remarque également que la diffusion étant permanente, il est difficile de prévoir des parfums élaborés et raffinés. Il faut des parfums immédiatement puissants avec un fort temps de rémanence.

Des dispositifs de diffusion d'un produit volatile dans l'habitacle d'un véhicule sont connus par les documents US-A-4 692 590 et DE-A-19 934 380.

Le but de la présente invention est de proposer un dispositif qui permet une diffusion variable et adaptée d'un parfum dans un véhicule, qui autorise le recours à des parfums raffinés haut de gamme, qui assure une diffusion immédiate avec un fort pouvoir de masquage, qui peut être adapté en fonction du véhicule et des occupants, qui supprime l'effet entêtant, qui est simple à mettre en oeuvre et qui est contrôlé par les occupants eux-mêmes.

A cet effet, le procédé de diffusion d'un produit volatil, dans l'habitacle d'un véhicule équipé d'un allume-cigare ayant au moins un élément chauffant, se caractérisé en ce qu'il consiste à disposer ledit produit volatil à proximité dudit élément chauffant de l'allume-cigare pour générer une diffusion rapide et efficace de ce produit volatil dès la mise en service de l'allume-cigare. Plus particulièrement, le procédé consiste à diffuser en permanence le produit volatil par action naturelle et à diffuser de façon puissante ce même produit volatil, à la demande, par manoeuvre de l'allume-cigare.

Le dispositif selon la présente invention comprend un allume-cigare muni d'au moins un élément de chauffage et de moyens de diffusion du produit volatil, moyens de diffusion qui peuvent comprendre un réservoir de liquide et des moyens d'absorption/diffusion reliés à ce réservoir.

Ce réservoir comprend éventuellement une valve de remplissage prévue pour coopérer avec une cartouche sous pression de produit volatil.

L'allume-cigare est muni d'une première résistance de chauffage et d'une seconde résistance de chauffage cette seconde résistance de chauffage étant reliée électriquement par une connexion à la première résistance et de puissance inférieure à cette première résistance, cette seconde résistance étant disposée à proximité immédiate des moyens de diffusion du produit volatil.

Selon un mode de réalisation, les moyens d'absorption/diffusion comprennent un élastomère poreux qui, lorsqu'il est comprimé exprime son contenu et qui, lorsqu' il est relâché, assure une aspiration.

Selon un autre mode de réalisation, les moyens d'absorption/diffusion comprennent une céramique.

La présente invention est maintenant décrite en regard des dessins annexés qui représentent un mode de réalisation particulier, non limitatif, les différentes figures montrant :
- figures 1A et 1B, deux vues en perspective du dispositif de diffusion de produits volatils selon la présente invention,
- figure 2, une vue en coupe du dispositif des figures 1A et 1B,
- figures 3A et 3B, deux vues en coupe des deux phases de fonctionnement,
- figure 4, une variante de réalisation à pastille, et
- figure 5, un mode de réalisation industriel.

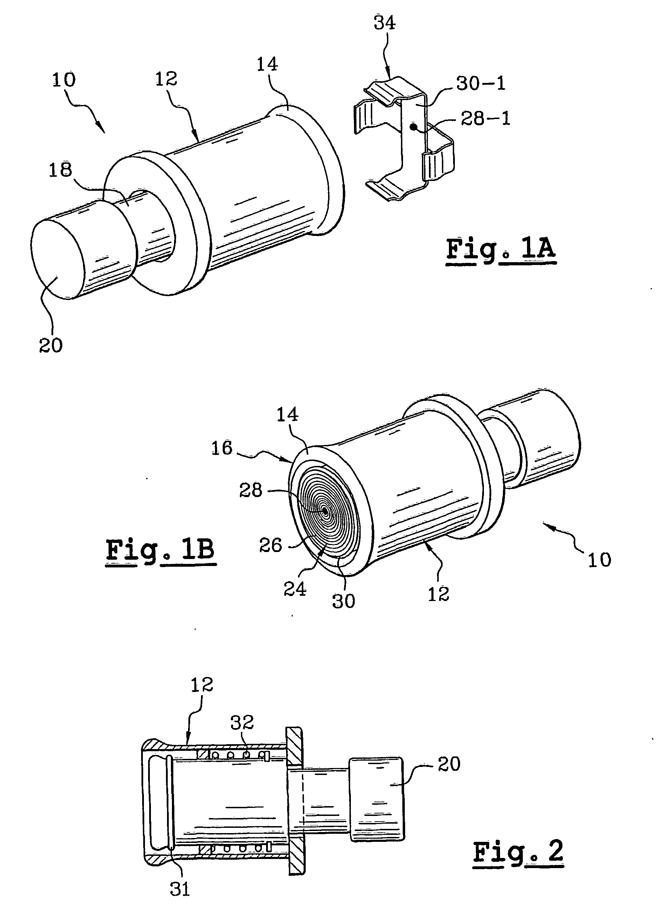

Sur les figures 1A et 1B, on a représenté un allume-cigare 10 tel qu'il se trouve dans la quasi-totalité des véhicules de tourisme et utilitaires. Dans certains véhicules particulièrement bien équipés ou haut de gamme, il est prévu plusieurs allume-cigares. Un tel moyen comprend de façon connue un capot 12 de protection, prévu pour venir s'emboîter dans un logement, non représenté, de profil adapté et ménagé dans une des consoles du véhicule. Une gorge 14 de retenue par encliquetage dans ce logement est prévue à l'extrémité 16 intérieure.

Un doigt 18 mobile est agencé de façon coulissante dans ce capot et peut prendre une position rétractée dans ledit capot et une position en saillie hors dudit capot.

Ce doigt comprend un pion 20 de préhension pour l'extrémité 22 extérieure et un élément 24 chauffant à son autre extrémité. Cet élément chauffant est généralement une résistance 26, enroulée en spirale du centre vers l'extérieur. La résistance est liée à un contact central 28 et un contact périphérique extérieur 30 Un joint 31, visible sur la figure 2, isole thermiquement et électriquement cet élément du doigt qui le porte.

La résistance peut, par ses contacts 28 et 30, venir se connecter, dans la position en saillie du doigt, avec des plots 28-1 et 30-1 d'alimentation électrique, disposés de façon adéquate dans la console, au sein du logement qui reçoit ledit allume-cigare.

Un élément 32 élastique de rappel ramène en permanence le doigt en position rétractée de sécurité dans laquelle la résistance n'est pas alimentée.

Des moyens 34 temporisés de retenue sont également prévus dans le logement pour tenir le doigt dans la position en saillie pendant la durée nécessaire à la montée en température de la résistance. Généralement, ces contacts électriques eux-mêmes assurent cette fonction car, sous l'effet de la chaleur dégagée par la résistance en chauffe, ils perdent leur capacité de maintien. L'utilisation d'un tel allume-cigare est simple. Il suffit pour l'occupant du véhicule de presser sur le pion 20 pour déplacer le doigt 18 par rapport au capot 12 qui, lui, est immobilisé dans le logement qui le reçoit.

L'élément 24 chauffant, plus particulièrement les contacts 28 et 30 viennent se connecter électriquement en appui sur les plots 28-1 et 30-1.

La résistance est soumise au passage du courant et s'échauffe par effet Joule. Les contacts 28-1 et 30-1 élastiques du logement tels que représentés, assurent la retenue du doigt par accrochage autour de l'élément chauffant. Lorsque la température s'élève, les capacités de retenue élastique s'affaiblissent. L'élément élastique de rappel 32 assure un effort de rappel sur le doigt supérieur à la capacité de retenue des contacts. Le doigt se libère et se rétracte par rapport au capot, l'alimentation électrique est interrompue et l'élément chauffant se retrouve protégé dans le capot.

L'occupant peut alors laisser l'allume-cigare en l'état s'il n'a plus l'utilité immédiate de cet accessoire et la résistance refroidit. L'occupant peut aussi retirer l'ensemble doigt et capot en exerçant une traction sur le pion 20. Il peut allumer sa cigarette en plaquant l'extrémité contre la résistance chauffée.

Dans le cas de la présente invention, il est prévu un allume-cigare adapté pour être introduit dans le même logement d'une des consoles et fonctionnant du point de vue du chauffage de la même façon.

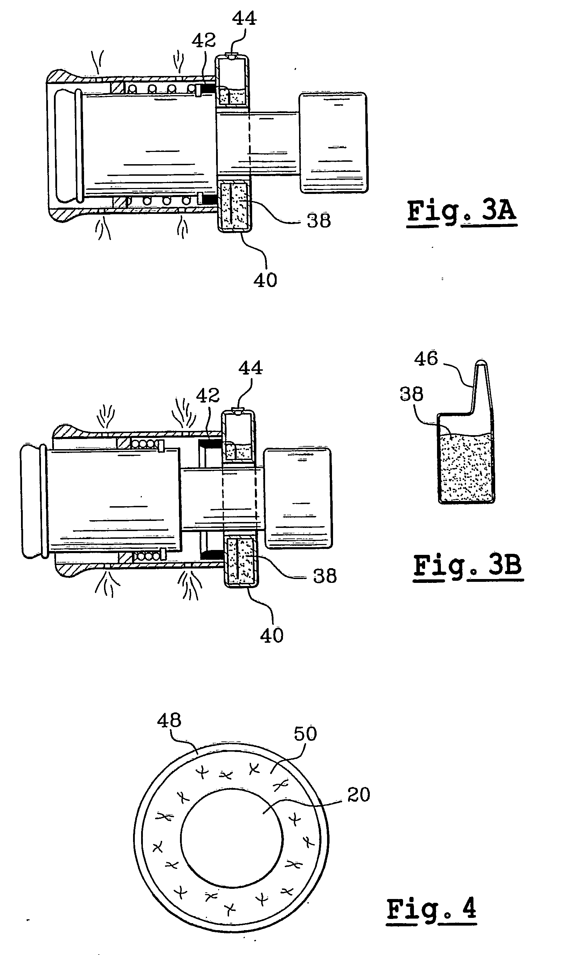

Par contre, sur les figures 3A et 3B qui représentent le dispositif de diffusion selon la présente invention, on constate la présence de moyens 36 de diffusion d'un liquide 38 volatil. Ces moyens comprennent en l'occurrence, un réservoir 40 de liquide, des moyens 42 d'absorption/diffusion reliés à ce réservoir.

Le réservoir 40 comprend une valve 44 de remplissage. Une telle valve est connue de l'homme de l'art et elle existe par exemple sur les briquets pour autoriser un remplissage de gaz sous forme liquide à partir d'une cartouche 46 sous pression.

Les moyens d'absorption/diffusion comprennent par exemple un élastomère poreux qui lorsqu'il est comprimé exprime son contenu et quand il est relâché assure une aspiration. En fonction des caractéristiques élastiques, du taux de porosité et de son coefficient de mouillabilité notamment, on peut décider de la quantité de liquide volatil absorbé et relâché à chaque pression. En effet, en disposant ces moyens d'absorption/diffusion entre le doigt et le capot, ceux-ci se trouvent comprimés lorsque le doigt est en position rétractée et en position dilatée lorsque le doigt est en saillie.

De ce fait, à chaque manoeuvre de l'allume-cigare, il se produit une diffusion du produit volatil. Cette diffusion est renforcée par le fait que la chaleur émise par la résistance assure une volatilisation plus rapide et plus efficace. Ceci procure une instantanéité de l'effet.

Si l'allume-cigare a été manoeuvré dans le but d'allumer une cigarette, l'odeur consécutive à la fumée va être masquée par le produit volatil.

L'allume-cigare peut aussi être manoeuvré uniquement pour diffuser le produit volatil et masquer une odeur désagréable venant de l'extérieur et introduite dans l'habitacle avec l'air de renouvellement. L'allume-cigare est alors laissé en place dans son logement.

La quantité absorbée doit être très faible et ceci est possible car la "pompe" telle que décrite peut diffuser quelques microlitres de liquide.

De ce fait, il est possible de diffuser un parfum élaboré et même des parfums de luxe attachés à des marques notoires. Dans ce cas, il peut y avoir un lien entre la dénomination du véhicule et la marque notoire du parfum diffusé. Mieux encore, le réservoir peut se prendre la forme du bouchon du flacon ou du flacon lui-même en sorte de rappeler la notoriété.

De même, il est possible de livrer un véhicule et de fournir le nécessaire pour obtenir l'ambiance recherchée.

On note aussi que dans un véhicule existant, il est possible de remplacer l'allume-cigare en place par un dispositif selon l'invention sans que cela ne requiert de transformations.

La description du mode de réalisation qui précède peut trouver de nombreuses variantes de réalisation pratique, comme celle de la figure 4.

Il est aussi possible en effet de prévoir simplement un réceptacle 48 et une ou des pastilles 50 déliquescentes à introduire dans ce réceptacle. Dans ce cas, l'effet obtenu est un peu différent en ce sens que les pastilles diffusent en continu une très faible quantité de parfum qui confère une faible capacité de masquage mais une ambiance agréable. Dès que l'allume-cigare est manoeuvré, la diffusion est renforcée temporairement avec un effet de masquage très supérieur.

Le réceptacle peut avoir une forme annulaire tout comme la pastille qu'il reçoit et venir en lieu et place du réservoir 40.

La pastille peut être réalisée en un matériau absorbant tel qu'une céramique poreuse à usage unique ou rechargeable.

De la même façon, selon une autre variante, il est possible de réaliser un allume-cigare en deux parties l'une chauffante à usage permanent et l'autre contenant le réservoir et les moyens de diffusion à usage unique non rechargeable, sous forme de cartouche jetable.

Lorsque la cartouche est vide, il suffit de remplacer la cartouche usagée par une neuve avec des moyens d'encliquetage comme pour des cartouches d'encres d'imprimantes, Dans ce cas, l'avantage est de disposer d'un nouveau volume de produit volatil et notamment de parfum mais aussi de pouvoir changer les moyens de diffusion qui peuvent se dégrader au cours du temps. L'agencement de cette cartouche sur l'allume-cigare peut prendre toute forme adaptée à condition que la production de chaleur de l'élément chauffant de l'allume-cigare permette une diffusion rapide du produit volatil.

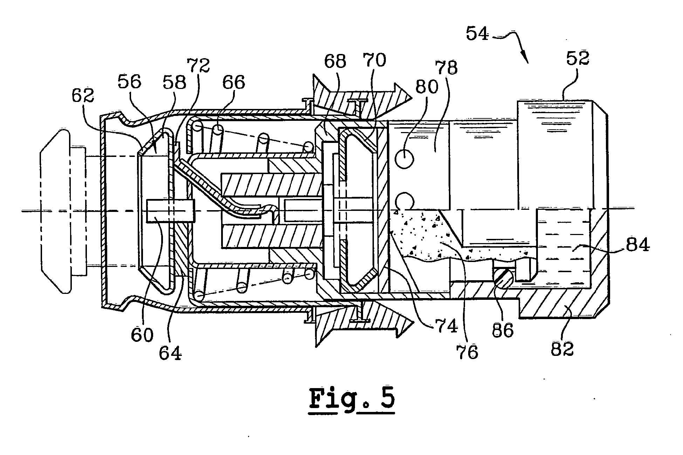

Sur la figure 5, on a représenté une variante industrielle de réalisation.

On retrouve les éléments essentiels du dispositif selon la présente invention.

Il est ainsi prévu des moyens 52 de préhension pour l'extrémité 54 extérieure et un élément 56 chauffant à son autre extrémité. Cet élément chauffant est généralement une résistance 58, enroulée en spirale du centre vers l'extérieur. La résistance est liée à un contact central 60 et un contact périphérique extérieur 62. Un joint 64 isole thermiquement et électriquement cet élément.

Un élément 66 élastique de rappel ramène en permanence le doigt avec sa résistance, en position rétractée de sécurité dans laquelle la résistance n'est pas alimentée.

Des moyens 68 temporisés de retenue sont également prévus dans le logement pour tenir le doigt dans la position en saillie pendant la durée nécessaire à la montée en température de la résistance.

En partie arrière, il est prévu une seconde résistance 70, alimentée en même temps que la première résistance en sorte de provoquer un échauffement distinct et maîtrisé. Cette résistance est isolée thermiquement, de préférence dans une enveloppe en céramique, et reliée à la première résistance par une connexion 72. Cette résistance a une puissance très inférieure à la première en sorte de provoquer une évaporation du parfum mais d'éviter une carbonisation. Dans certains cas, la composition du parfum est délicate et les produits organiques qui la constituent, peuvent dégager une odeur non souhaitable en cas de trop forte température.

Un disque 74 de diffusion peut être disposé devant la résistance. Ce disque reçoit la chaleur produite par la résistance et permet une homogénéisation de la chaleur à diffuser, Ce disque est optionnel en ce sens que la résistance, montée en parallèle, peut avoir une puissance moindre et parfaitement adaptée.

Ce disque a aussi un autre avantage, il évite le contact direct de la composition du parfum avec la résistance

Un diffuseur 76, en céramique poreuse dans ce mode de réalisation, est plaqué contre le disque 74 de diffusion. Ce diffuseur est protégé par une jupe 78 munie d'évents 80, de diffusion.

Le réservoir 82, amovible, contenant le parfum 84, est rapporté de façon étanche sur un joint 86 d'étanchéité, du type torique.

Avantageusement, chaque réservoir est équipé d'un opercule, non représenté, qui assure l'obturation dudit réservoir et qui se déchire lorsque ce réservoir est mis en place.

Le fonctionnement est strictement identique pour l'occupant d'un véhicule à celui présenté ci-avant dans sa la description schématique des figures 3A et 3B.

## Revendications

1. Dispositif de diffusion d'un produit (84) volatil, dans l'habitacle d'un véhicule équipé d'un allume-cigare ayant au moins un élément chauffant et des moyens de diffusion (74, 76) dudit produit (84) volatil, **caractérisé en ce que** cet allume-cigare est muni d'une première résistance (58) de chauffage et d'une seconde résistance de chauffage (70), cette seconde résistance de chauffage étant reliée électriquement par une connexion (72) à la première (58) résistance et de puissance inférieure à cette première résistance, cette seconde résistance étant disposée à proximité immédiate des moyens (74,76) de diffusion du produit (84) volatil.

2. Dispositif selon la revendication 1, **caractérisé en ce que** les moyens de diffusion comprennent un réservoir (82) de liquide et des moyens (74,76) d'absorption/diffusion reliés à ce réservoir.

3. Dispositif selon la revendication 2, **caractérisé en ce qu'**il comprend un disque (74) de diffusion, interposé entre la seconde résistance (70) et les moyens de diffusion (76).

4. Dispositif selon la revendication 2 ou 3, **caractérisé en ce qu'**il comprend une jupe (78) de protection des moyens (76) de diffusion, munie d'évents (80).

5. Dispositif selon l'une quelconque des revendications 2, 3 ou 4, **caractérisé en ce que** le réservoir (82) est amovible et rapporté en contact étanche avec un joint (86).

6. Dispositif selon l'une quelconque des revendications 2, 3, 4 ou 5, **caractérisé en ce que** le réservoir (82) comprend un opercule assurant l'obturation dudit réservoir et qui se déchire lorsque ce réservoir est mis en place.

## Claims

1. A device for diffusing a volatile product (84) in the cabin of a vehicle equipped with a cigar lighter having at least one heating element and means (74, 76) of diffusing said volatile product (84), **characterised in that** this cigar lighter is provided with a first heating element (58) and a second heating element (70), this second heating element being electrically connected by a connection (72) to the first element (58) and with a power less than this first element, this second element being disposed in the immediate vicinity of the means (74, 76) of diffusing the volatile product (84).

2. A device according to claim 1, **characterised in that** the diffusion means comprises a reservoir (82) of liquid and an absorption/diffusion means (74, 76) connected to this reservoir.

3. A device according to claim 2, **characterised in that** it comprises a diffusion disc (74), interposed between the second element (70) and the diffusion means (76).

4. A device according to claim 2 or 3, **characterised in that** it comprises a skirt (78) for protecting the diffusion means (76), provided with vents (80).

5. A device according to any one of claims 2, 3 or 4, **characterised in that** the reservoir (82) is removable and attached in sealed contact with a gasket (86).

6. A device according to any one of claims 2, 3, 4 or 5, **characterised in that** the reservoir (82) comprises a membrane seal providing closure of said reservoir and which tears when this reservoir is fitted.

## Patentansprüche

1. Vorrichtung zur Diffusion eines flüchtigen Produktes (84) in eine Fahrzeugkabine, die mit einem Zigarettenanzünder ausgestattet ist, welcher mindestens ein Heizelement und Mittel (74, 76) zur Diffusion des flüchtigen Produktes (84) aufweist, **dadurch** gekennzeichet, dass dieser Zigarettenanzünder mit einem ersten Heizwiderstand (58) und mit einem zweiten Heizwiderstand (70) versehen ist, wobei dieser zweite Heizwiderstand über eine Verbindung (72) mit dem ersten (58) Widerstand elektrisch verbunden ist und eine geringere Leistung als dieser erste Widerstand aufweist, wobei dieser zweite Widerstand in unmittelbarer Nähe der Mittel (74, 76) zur Diffusion des flüchtigen Produktes (84) angeordnet ist.

2. Vorrichtung nach Anspruch 1, **dadurch gekennzeichnet, dass** die Diffusionsmittel einen Flüssigkeitsbehälter (82) und mit diesem Behälter in Verbindung stehende Mittel (74, 76) zur Absorption/Diffusion umfassen.

3. Vorrichtung nach Anspruch 2, **dadurch gekennzeichnet, dass** sie eine Diffusionsscheibe (74) umfasst, die zwischen dem zweiten Widerstand (70) und den Diffusionsmitteln (76) angeordnet ist.

4. Vorrichtung nach Anspruch 2 oder 3, **dadurch gekennzeichnet, dass** sie einen Schutzmantel (78) zum Schutz der Diffusionsmittel (76) umfasst, welcher mit Lüftungsöffnungen (80) versehen ist.

5. Vorrichtung nach einem der Ansprüche 2, 3 oder 4, **dadurch gekennzeichnet, dass** der Behälter (82) abnehmbar ist und in dichtem Kontakt mit einer Dichtung (86) angesetzt ist.

6. Vorrichtung nach einem der Ansprüche 2, 3, 4 oder 5, **dadurch gekennzeichnet, dass** der Behälter (82) ein Verschlusselement umfasst, das den Behälter verschließt und das zerreißt, wenn der Behälter eingesetzt wird.
